# FASCICULE DE BREVET EUROPEEN

(11) **EP 4 049 289 B1**
(45) Date de publication et mention de la délivrance du brevet: **01.10.2025**
(21) Numéro de dépôt: 20790339.4
(22) Date de dépôt: 21.10.2020
(51) Int. Cl.: G16H 50/50, A61C 7/00, A61B 5/103, G06T 15/04, G06T 5/00

(54) **PROGRAMME D'ORDINATEUR DE GENERATION D'UNE IMAGE DENTAIRE**
COMPUTERPROGRAMM ZUM ERZEUGEN EINES DENTALEN BILDES
COMPUTER PROGRAM FOR GENERATING A DENTAL IMAGE

(30) Priorité: 22.10.2019 FR 1911793
(43) Date de publication de la demande: 31.08.2022
(73) Titulaire: Dental Monitoring, 75017 Paris (FR)
(72) Inventeur: SALAH, Philippe, 75020 Paris (FR); PELLISSARD, Thomas, 75004 Paris (FR); GHYSELINCK, Guillaume, 59169 Cantin (FR); MISRACHI, Laura, 75006 Paris (FR)
(74) Mandataire: Novagraaf Group
(86) Numéro de dépôt international: PCT/EP2020/079580
(87) Numéro de publication internationale: WO 2021/078778

(56) Documents cités:
- EP-A1- 3 050 534
- WO-A1-2018/112427
- CN-A- 107 518 952
- US-A1- 2016 038 092
- US-A1- 2018 168 781
- US-A1- 2018 263 733
- PAUL UPCHURCH ET AL: "Deep Feature Interpolation for Image Content Changes", ARXIV.ORG, CORNELL UNIVERSITY LIBRARY, 201 OLIN LIBRARY CORNELL UNIVERSITY ITHACA, NY 14853, 17 November 2016 (2016-11-17), XP080732363, DOI: 10.1109/CVPR.2017.645
- LI YU ET AL: "Unsupervised Local Facial Attributes Transfer Using Dual Discriminative Adversarial Networks", 2018 IEEE INTERNATIONAL CONFERENCE ON MULTIMEDIA AND EXPO (ICME), IEEE, 23 July 2018 (2018-07-23), pages 1 - 6, XP033417638, DOI: 10.1109/ICME.2018.8486562

## Description

### Domaine technique

La présente invention concerne la génération d'images d'arcades dentaires. Elle se rapporte en particulier à un procédé pour visualiser sur une image, de manière réaliste, l'effet d'un évènement dentaire susceptible d'affecter l'arcade dentaire d'un bénéficiaire, par exemple d'un patient en cours de traitement orthodontique.

### Etat de la technique

PCT/EP2019/068558 décrit un procédé dans lequel des modèles tridimensionnels numériques d'arcades dentaires, et notamment à des scans réalisés par les professionnels de soins dentaires, sont déformés pour simuler (c'est-à-dire sont adaptés pour reproduire artificiellement) une situation dentaire que l'on anticipe à un instant de simulation passé ou que l'on prévoit à un instant de simulation futur. Les modèles déformés sont utilisés pour créer des vues hyperréalistes équivalentes à des photos.

WO 2018/112427 A1 divulgue l'utilisation d'un réseau de neurones pour identifier une zone d'intérêt sur une image dentaire.

US 2018/263733 A1 divulgue un procédé pour intégrer plus étroitement les modèles 3D d'un patient dans des images 2D.

Les personnes doivent donc se déplacer chez l'orthodontiste pour réaliser les modèles de leurs arcades dentaires. Le domaine d'application de ce procédé est donc limité.

Il existe donc un besoin pour un procédé de génération d'images d'arcade dentaire simulant l'effet d'un évènement dentaire et qui n'imposerait pas la génération d'un modèle.

Un but de l'invention est de répondre à ce besoin.

### Résumé de l'invention

L'invention concerne un programme d'ordinateur comprenant des instructions de code de programme pour l'exécution d'un procédé de génération d'une image d'une arcade dentaire d'un bénéficiaire, dite « image modifiée », selon la revendication 1. Les façon de la réaliser préférentielles sont définies dans les revendications dépendantes.

**Selon un premier aspect principal,** l'invention concerne un programme d'ordinateur comprenant des instructions de code de programme pour l'exécution d'un procédé de génération d'une image d'une arcade dentaire d'un bénéficiaire, dite « image modifiée », ledit procédé comportant les étapes successives suivantes :
a) à un instant d'acquisition, acquisition d'une photo représentant ladite arcade dentaire, dite « image d'origine » ;
b) traitement de l'image d'origine pour qu'elle représente une information discriminante, de préférence un contour de l'arcade dentaire ;
c) soumission de l'image d'origine à un réseau de neurones, dit « réseau de neurones de simulation », entrainé pour simuler, sur l'image d'origine, l'effet d'un évènement dentaire, afin d'obtenir l'image modifiée ;
   l'événement dentaire étant choisi parmi un écoulement de temps dans le cadre d'un traitement orthodontique ou non orthodontique, dans le cadre d'une pathologie ou dans le cadre d'un bruxisme, une pose d'un organe dentaire sur l'arcade dentaire, un écoulement du temps en l'absence de traitement, et les combinaisons de ces évènements dentaires;
d) de préférence, traitement de l'image modifiée pour la rendre hyperréaliste ;
e) de préférence,

- présentation de l'image modifiée, de préférence au moins au bénéficiaire et/ou à un professionnel des soins dentaires ; et/ou
- sélection d'un appareil orthodontique, par ordinateur et/ou par le bénéficiaire et/ou par un professionnel des soins dentaires, en fonction de l'image modifiée, puis, de préférence, fabrication dudit appareil orthodontique.

Comme on le verra plus en détail dans la suite de la description, un programme d'ordinateur comprenant des instructions de code de programme pour l'exécution d'un procédé de génération selon l'invention ne nécessite pas de générer un modèle de l'arcade dentaire du bénéficiaire. De manière remarquable, toute personne, en cours de traitement ou non, peut obtenir une simulation de l'effet de l'évènement dentaire sur ses dents, sans avoir à se déplacer chez l'orthodontiste.

En particulier, l'image modifiée représente l'arcade telle que simulée après application de l'évènement dentaire. Le bénéficiaire peut ainsi bénéficier d'une simulation qui lui permet de bien mesurer l'impact visuel de l'évènement dentaire.

Un programme d'ordinateur comprenant des instructions de code de programme pour l'exécution d'un procédé de génération selon l'invention peut encore comporter une ou plusieurs des caractéristiques optionnelles suivantes :
- l'évènement dentaire est choisi parmi un écoulement de temps dans le cadre d'un traitement orthodontique ou non orthodontique, dans le cadre d'une pathologie ou dans le cadre d'un bruxisme, une pose d'un organe dentaire sur l'arcade dentaire, un écoulement du temps en l'absence de traitement, et les combinaisons de ces évènements dentaires ;
- l'information discriminante est choisie dans le groupe constitué par un information de contour, une information de couleur, une information de densité, une information de distance, une information de brillance, une information de saturation, une information sur les reflets et des combinaisons de ces informations ;
- le réseau de neurones de simulation est entrainé au moyen d'un procédé d'entrainement selon l'invention, décrit ci-après ;
- le cycle des étapes 1) à 4) est répété, les premières conditions d'observation étant modifiées à la fin de chaque étape 4) ;
- à l'étape a), on acquiert la photo de manière extra-orale, de préférence avec un téléphone portable, le bénéficiaire portant de préférence un écarteur dentaire ;
- le procédé comporte une étape d) de traitement de l'image modifiée pour la rendre hyperréaliste, l'étape d) comportant les étapes suivantes :
   d0) pour chaque photo, dite « photo de texturation », représentant une arcade dentaire d'un ensemble comportant plus de 1 000 photos de texturation,
   traitement de la photo de texturation, de préférence comme à l'étape b), de manière à obtenir une image, dite « image de texturation », représentant un contour ;
   d1) création d'une base d'apprentissage dite « de texturation » constituée d'enregistrements, dits « enregistrements de texturation », chaque enregistrement de texturation comportant une photo de texturation et l'image de texturation obtenue par traitement de ladite photo de texturation à l'étape d0) ;
   d2) entrainement d'un réseau de neurones, dit « réseau de neurones de texturation », au moyen de la base d'apprentissage de texturation ;
   d3) soumission de l'image modifiée au réseau de neurones de texturation entrainé, de manière à obtenir une image modifiée hyperréaliste ;
- l'évènement dentaire est un écoulement de temps depuis l'instant d'acquisition jusqu'à un instant de simulation antérieur ou postérieur à l'instant d'acquisition de plus de 1 jour, et dans lequel à l'étape e), l'image modifiée est présentée au bénéficiaire afin de lui montrer une situation dentaire déterminée, c'est-à-dire simulé, audit instant de simulation ;
- avant l'étape c), on détermine l'évènement dentaire en précisant un instant de simulation, et/ou un paramètre d'un traitement appliqué au bénéficiaire et/ou un paramètre d'un appareil orthodontique porté par le bénéficiaire, et/ou un paramètre fonctionnel du bénéficiaire, et/ou un paramètre anatomique du bénéficiaire autre que les paramètres de positionnement de ses dents, et/ou un âge, ou une tranche d'âge, et/ou un sexe dudit bénéficiaire ;
- les étapes a) à e) sont répétées en boucle, avec des images d'origine acquises successivement, chaque cycle durant moins de 5 s, de préférence moins de 1 s.

Pour le programme de la présente invention, est préférentiellement utilisé un procédé d'entrainement d'un réseau de neurones, ledit procédé comportant, pour chacun d'une pluralité de modèles tridimensionnels numériques d'arcades dentaires « historiques », dit « modèles historiques », les étapes 1) à 3) successives suivantes :
1) acquisition d'une première vue du modèle historique dans des premières conditions d'observations et, si ladite première vue ne représente pas une information discriminante de l'arcade dentaire historique, dite « première information discriminante », de préférence ne représente pas un contour de l'arcade dentaire historique, dit « premier contour », traitement de la première vue de manière qu'elle représente ladite première information discriminante, de préférence un premier contour ;
2) modification du modèle historique, par exemple par déformation et/ou ajout d'un organe dentaire, de manière à reproduire l'effet d'un évènement dentaire sur ladite arcade dentaire historique ;
3) acquisition d'une deuxième vue du modèle historique dans des deuxièmes conditions d'observations identiques aux premières conditions d'observations et, si ladite deuxième vue ne représente pas ladite information discriminante de l'arcade dentaire historique, dite « deuxième information discriminante », en particulier si ladite deuxième vue ne représente pas un contour de l'arcade dentaire historique, dit « deuxième contour », traitement de la deuxième vue de manière qu'elle représente ladite deuxième information discriminante, de préférence un deuxième contour, et création d'un enregistrement historique avec les première et deuxième vues ;
puis, avec l'ensemble des enregistrements historiques créés pour l'ensemble des modèles historiques :
4) introduction, en entrée et en sortie du réseau de neurones, desdites premières et deuxièmes vues, respectivement, de manière à entrainer ledit réseau de neurones à transformer une vue d'entrée représentant une arcade dentaire d'analyse en une vue de sortie représentant ladite arcade dentaire d'analyse après application dudit évènement dentaire.

L'observation du modèle historique dans les mêmes conditions d'observations permet d'obtenir très facilement des première et deuxième vues parfaitement en registre. Il n'y a pas de recadrage nécessaire.

Dans un mode de réalisation,
- à l'étape 1), on acquiert plus de 10, plus de 100, plus de 1 000, plus de 10 000 premières vues dans des premières conditions d'observations à chaque fois différentes, puis,
- à l'étape 3), pour chaque première vue acquise dans des premières conditions d'observation, on acquiert une deuxième vue dans des deuxièmes conditions d'observation identiques aux dites premières conditions d'observation et on crée un enregistrement historique avec lesdites première et deuxième vues.

De préférence, le réseau de neurones de simulation utilisé à l'étape c) est entrainé avec un procédé d'entrainement selon l'invention.

Tout ordinateur peut être envisagé, notamment un téléphone, un PC, un serveur, ou une tablette.

### Définitions

Un « traitement orthodontique » est tout ou partie d'un traitement destiné à modifier la configuration d'une arcade dentaire.

Par « organe dentaire », on entend tout dispositif destiné à être porté par l'arcade dentaire, et en particulier un appareil orthodontique, une couronne, un implant, un bridge, ou une facette.

Une « situation dentaire » définit un ensemble de caractéristiques relatives à une arcade d'un patient à un instant, par exemple la position des dents, leur forme, leur couleur, la position d'un appareil orthodontique, etc. à cet instant.

Un « bénéficiaire » est une personne pour laquelle un procédé selon l'invention est mis en œuvre, indépendamment du fait que cette personne suive un traitement orthodontique ou non.

Par « professionnel de soins dentaires », on entend toute personne qualifiée pour prodiguer des soins dentaires, ce qui inclut en particulier un orthodontiste et un dentiste.

Par « ordinateur », on entend tout appareil électronique ayant des capacités de traitement informatique.

Un « écarteur » (« retractor » en anglais), ou « écarteur dentaire », est un dispositif destiné à retousser les lèvres. Il comporte un rebord supérieur et un rebord inférieur s'étendant autour d'une ouverture d'écarteur. En position de service, les lèvres supérieure et inférieure du patient sont en appui sur les rebords supérieur et inférieur, respectivement. L'écarteur est configuré de manière à écarter élastiquement l'une de l'autre les lèvres supérieure et inférieure de manière à dégager les dents visibles à travers l'ouverture. Un écarteur permet ainsi d'observer les dents sans être gêné par les lèvres. Les dents ne reposent cependant pas sur l'écarteur, de sorte que le patient peut, en tournant la tête par rapport à l'écarteur, modifier les dents qui sont visibles à travers l'ouverture de l'écarteur. Il peut aussi modifier l'écartement entre les arcades. En particulier, un écarteur n'appuie pas sur les dents de manière à écarter les deux mâchoires l'une de l'autre. De préférence, l'écarteur comporte des oreilles d'écartement des joues, ce qui permet d'acquérir, à travers l'ouverture d'écarteur, des photos des faces vestibulaires des dents au fond de la bouche, comme les molaires.

Par « modèle », on entend un modèle tridimensionnel numérique. Un modèle est constitué d'un ensemble de voxels.

Dans un souci de clarté, on distingue classiquement le « découpage » d'un modèle d'arcade en « modèles élémentaires » et la « segmentation » d'une image en « zones élémentaires ». Les modèles élémentaires et les zones élémentaires sont des représentations, en 3D ou en 2D respectivement, d'un élément d'une scène réelle, par exemple d'une dent.

Les « conditions d'observation » d'un modèle précisent la position dans l'espace, l'orientation dans l'espace et la calibration, par exemple les valeurs de l'ouverture de diaphragme et/ou du temps d'exposition et/ou de la distance focale et/ou de la sensibilité, d'un appareil d'acquisition d'images virtuel, relativement à ce modèle.

La « calibration » d'un appareil d'acquisition est constituée par l'ensemble des valeurs des paramètres de calibration. Un paramètre de calibration est un paramètre intrinsèque à l'appareil d'acquisition (à la différence de sa position et de son orientation) dont la valeur influence l'image acquise. Par exemple, l'ouverture de diaphragme est un paramètre de calibration qui modifie la profondeur de champ. Le temps d'exposition est un paramètre de calibration qui modifie la luminosité (ou « l'exposition ») de l'image. La distance focale est un paramètre de calibration qui modifie l'angle de vue, c'est-à-dire le degré de « zoom ». La « sensibilité » est un paramètre de calibration qui modifie la réaction du capteur d'un appareil d'acquisition numérique à la lumière incidente.

Une observation d'un modèle, dans des conditions d'observations déterminées, est appelée une « vue ».

Une « image » est une représentation, en deux dimensions et formée de pixels, d'une arcade. Une « photo » est donc une image particulière, classiquement en couleur, prise avec un appareil photo. Par « appareil photo », on entend tout appareil permettant de prendre une photo, ce qui inclut une caméra, un téléphone portable, une tablette ou un ordinateur. Une vue est un autre exemple d'image.

Par « photo d'une arcade », « représentation d'une arcade », « scan d'une arcade », « modèle d'une arcade », « image d'une arcade », « vue d'une arcade » ou « contour d'une arcade », on entend une photo, une représentation, un scan, un modèle, une image, une vue ou un contour de tout ou partie de ladite arcade dentaire, de préférence d'au moins 2, de préférence au moins 3, de préférence au moins 4 dents.

Par « évènement dentaire » on entend un fait susceptible de modifier une arcade dentaire, par exemple le port d'un appareil orthodontique ou le simple écoulement du temps.

Une "information discriminante" est une information caractéristique qui peut être extraite d'une image ( *"image feature* "), classiquement par un traitement informatique de cette image.

Une information discriminante peut présenter un nombre variable de valeurs. Par exemple, une information de contour peut représenter une probabilité qu'un pixel appartient à un contour, et prendre par exemple une valeur comprise entre 0 et 255, 0 étant une probabilité très faible que le pixel appartient à un contour et 255 une probabilité très élevée. Dans un mode de réalisation, l'information discriminante est seuillée, c'est-à-dire que seule l'information discriminante qui dépasse une valeur de seuil prédéterminée est représentée. Par exemple, dans l'exemple précédent d'une information de contour, seules les valeurs supérieures à 200 sont représentées. Une information de brillance peut prendre un grand nombre de valeurs. Le traitement de l'image permet d'extraire et de quantifier l'information discriminante.

L'information discriminante peut être représentée dans une image, parfois appelée « carte ». Une carte est ainsi le résultat d'un traitement d'une image afin de faire apparaître l'information discriminante. Par exemple, le contour des dents et des gencives peut être la représentation de l'information de contour d'une image d'origine. La représentation d'un contour est donc la représentation de l'information de contour sous la forme d'une image.

Un « contour » est une ligne ou un ensemble de lignes qui délimite(nt) un objet et de préférence les éléments constitutifs de cet objet. Par exemple, le contour d'une dentition pour être une ligne qui définit les limites extérieures de cette dentition. De préférence, il comprend en outre les lignes qui définissent les limites entre les dents adjacentes. De préférence, le contour de la dentition est donc constitué de l'ensemble des contours des dents qui constituent cette dentition.

Un contour représenté sur une image peut être complet, et donc fermé sur lui-même, ou incomplet.

Le contour d'une arcade dentaire représente de préférence le contour de la dentition de cette arcade et, de préférence, les contours de chaque dent représentée, dits « contours élémentaires ».

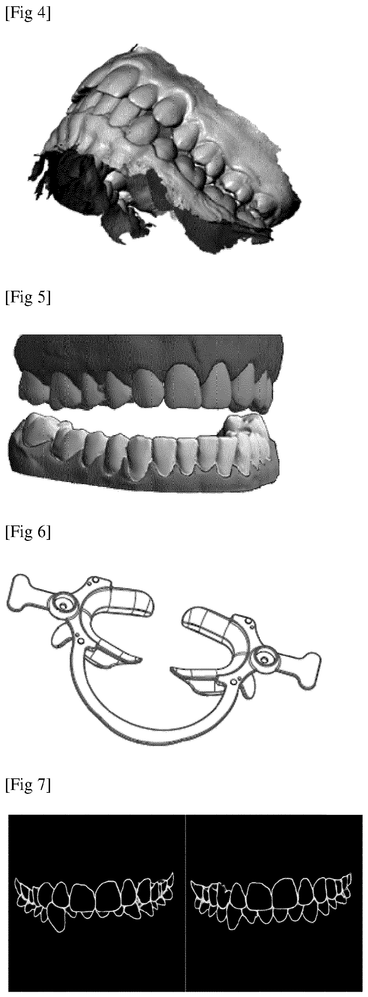

Dans un procédé de l'invention, les traitements appliqués à des images sont de préférence configurés pour faire apparaître, de préférence isoler les mêmes contours, de préférence des contours comportant, de préférence sensiblement constitués par l'ensemble des contours élémentaires des dents représentées, comme sur la figure 7.

Un « réseau de neurones » ou « réseau neuronal artificiel » est un ensemble d'algorithmes bien connu de l'homme de l'art. Pour être opérationnel, un réseau de neurones doit être entrainé par un processus d'apprentissage appelé *« deep learning »,* à partir d'une base d'apprentissage.

Une « base d'apprentissage » est une base d'enregistrements informatiques adaptée à l'entrainement d'un réseau de neurones. La qualité de l'analyse réalisée par le réseau de neurones dépend directement du nombre d'enregistrements de la base d'apprentissage. Classiquement, la base d'apprentissage comporte plus de 10 000 enregistrements.

L'entrainement d'un réseau de neurones est adapté au but poursuivi et ne pose pas de difficulté particulière à l'homme de l'art.

L'entrainement d'un réseau de neurones consiste à le confronter à une base d'apprentissage contenant des informations sur les deux types d'objet que le réseau de neurones doit apprendre à faire « correspondre », c'est-à-dire à connecter l'un à l'autre.

L'entrainement peut se faire à partir d'une base d'apprentissage « pairée » ou « avec paires », constituée d'enregistrements « de paires », c'est-à-dire comportant chacun un premier objet d'un premier type pour l'entrée du réseau de neurones, et un deuxième objet correspondant, d'un deuxième type, pour la sortie du réseau de neurones. On dit aussi que l'entrée et la sortie du réseau de neurones sont « pairées ». L'entrainement du réseau de neurones avec toutes ces paires lui apprend à fournir, à partir d'un objet quelconque du premier type, un objet correspondant du deuxième type.

Par exemple, chaque enregistrement de la base d'apprentissage peut comporter une première vue d'un modèle d'une arcade dentaire et une deuxième vue de ce modèle, après survenue d'un évènement dentaire. Après avoir été entrainé avec cette base d'apprentissage, le réseau de neurones pourra transformer une vue d'un modèle d'une arcade dentaire en une vue de ce modèle modifiée pour simuler l'effet d'un dit évènement dentaire.

L'article « Image-to-Image Translation with Conditional Adversarial Networks » de Phillip Isola Jun-Yan Zhu, Tinghui Zhou, Alexei A. Efros, Berkeley AI Research (BAIR) Laboratory, UC Berkeley, illustre l'utilisation d'une base d'apprentissage pairée.

Dans la présente description, les qualificatifs « historique », « d'origine », « de texturation », « de simulation » et « d'analyse » sont utilisés à des fins de clarté.

Il faut interpréter "comprenant " ou "comportant " ou "présentant " de manière non restrictive, sauf indication contraire.

### Brève description des figures

D'autres caractéristiques et avantages de l'invention apparaîtront encore à la lecture de la description détaillée qui va suivre et à l'examen du dessin annexé dans lequel :
- [Fig 1] la figure 1 représente, schématiquement, les différentes étapes d'un mode de réalisation préféré d'un procédé de génération d'images selon l'invention ;
- [Fig 2] la figure 2 représente schématiquement, les différentes étapes d'un mode de réalisation préféré d'un procédé d'entrainement selon l'invention ;
- [Fig 3] la figure 3 représente un exemple d'une photo acquise à l'étape a), et de l'image modifiée obtenue au moyen d'un procédé selon l'invention entrainé avec des enregistrements comme celui de la figure 7 ;
- [Fig 4] la figure 4 représente un exemple d'un modèle d'une arcade dentaire ;
- [Fig 5] la figure 5 représente une vue d'un modèle d'une arcade dentaire ;
- [Fig 6] la figure 6 représente un exemple d'écarteur ;
- [Fig 7] la figure 7 représente un exemple d'enregistrement utilisé pour entrainer le réseau de neurones de simulation afin de simuler l'évènement « traitement orthodontique avec un appareil orthodontique à arc et attaches », les images de gauche et de droite étant introduites en entrée et en sortie du réseau de neurones de simulation, respectivement ;
- [Fig 8] la figure 8 représente un exemple de modèle découpé en modèles de dents, les autres éléments de l'arcade n'étant pas représentés.

### Description détaillée

La description détaillée qui suit est celle de modes de réalisation préférés, mais n'est pas limitative.

En particulier, elle décrit l'utilisation d'une information discriminante qui est une information de contour. L'information discriminante pourrait cependant être d'une autre nature.

### Entrainement d'un réseau de neurones

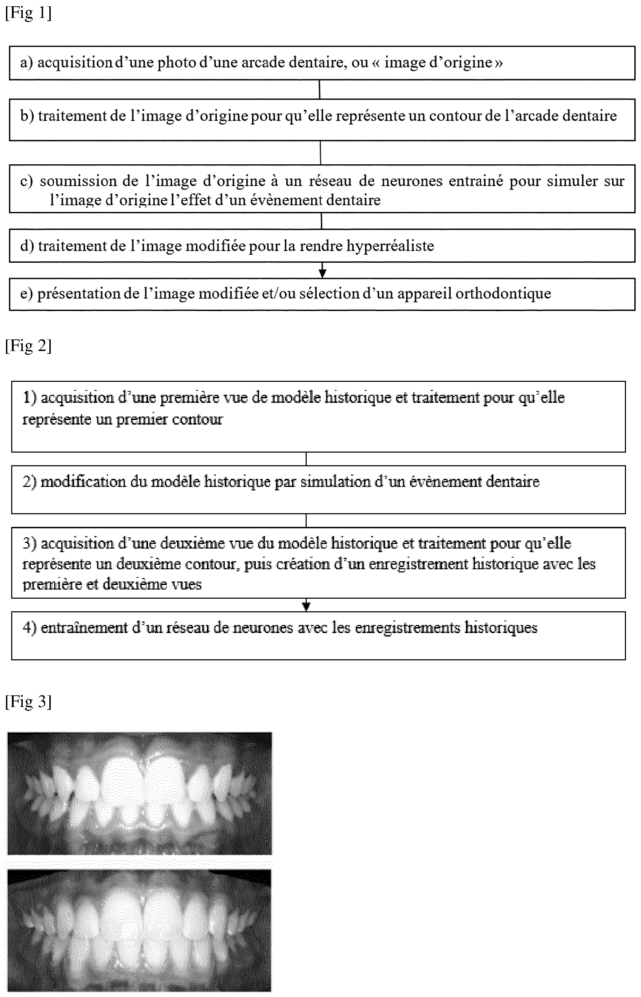

Le procédé d'entrainement d'un réseau de neurones selon l'invention comporte de préférence les étapes 1) à 4) (figure 1).

Les étapes 1) à 3) de ce procédé permettent avantageusement de multiplier les enregistrements utilisés à l'étape 4).

Préalablement à ces étapes, on génère un, de préférence plusieurs, de préférence plus de 100, de préférence plus de 1000, de préférence plus de 10 000 modèles historiques.

Chaque modèle historique représente une arcade dentaire d'un individu dit « historique ».

Le modèle historique peut être préparé à partir de mesures effectuées sur les dents de l'individu historique ou sur un moulage de ses dents, par exemple un moulage en plâtre.

Le modèle historique est de préférence obtenu à partir d'une situation réelle, de préférence créé avec un scanner 3D. Un tel modèle, dit « 3D », peut être observé selon un angle quelconque (Figure 4).

De préférence, on découpe le modèle historique. En particulier, de préférence, pour chaque dent, on définit, à partir du modèle historique, un modèle de ladite dent, ou « modèle de dent ».

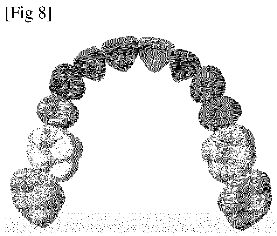

Le découpage d'un modèle historique d'une arcade dentaire en modèles de dent est une opération classique par laquelle le modèle de l'arcade est découpé afin de délimiter la représentation d'une ou plusieurs des dents dans le modèle (Figure 8).

Le modèle historique peut être découpé manuellement par un opérateur, à l'aide d'un ordinateur, ou être découpé automatiquement, par un ordinateur, de préférence en mettant en œuvre un dispositif d'apprentissage profond, de préférence un réseau de neurones. En particulier, les modèles de dents peuvent être définis comme décrit, par exemple, dans la demande internationale PCT/EP2015/074896.

Dans le modèle historique, un modèle de dent est de préférence délimité par un bord gingival qui peut être décomposé en un bord gingival intérieur (du côté de l'intérieur de la bouche par rapport à la dent), un bord gingival extérieur (orienté vers l'extérieur de la bouche par rapport à la dent) et deux bords gingivaux latéraux.

De manière similaire, on peut définir, à partir du modèle historique, d'autres modèles élémentaires que les modèles de dent, et notamment des modèles pour la langue, et/ou la bouche, et/ou les lèvres, et/ou les mâchoires, et/ou la gencive, et/ou un organe dentaire, notamment d'un appareil orthodontique.

Dans un mode de réalisation, le modèle historique est théorique, c'est-à-dire ne correspond pas à une situation réelle. En particulier, le modèle historique peut être créé par assemblage d'un ensemble de modèles de dents choisis dans une bibliothèque numérique. L'agencement des modèles de dent est déterminé pour que le modèle historique soit réaliste, c'est-à-dire corresponde à une situation qui aurait pu se rencontrer chez un individu. En particulier, les modèles de dents sont disposés suivant un arc, en fonction de leur nature, et orientés de manière réaliste. L'utilisation d'un modèle historique théorique permet avantageusement de simuler des arcades dentaires présentant des caractéristiques rares.

Un modèle historique fournit de préférence une information sur le positionnement des dents avec une erreur inférieure à 5/10 mm, de préférence inférieure à 3/10 mm, de préférence inférieure à 1/10 mm.

Un modèle historique est par exemple du type .stl ou .Obj, .DXF 3D, IGES, STEP, VDA, ou Nuages de points. Avantageusement, un tel modèle, dit « 3D », peut être observé selon un angle quelconque.

Pour chaque modèle historique, on procède selon les étapes 1) à 4).

**A l'étape 1),** on acquiert une première vue du modèle historique dans des premières conditions d'observations, c'est-à-dire en plaçant virtuellement un appareil d'acquisition d'image virtuel dans ces premières conditions d'observations, puis en acquérant la première vue avec cet appareil ainsi configuré.

La première vue est de préférence une vue extra-orale, par exemple une vue correspondant à une photo qui aurait été prise face au patient, de préférence avec un écarteur.

La figure 6 représente un exemple d'écarteur.

Si la première vue ne représente pas un contour, ou ne permet pas de l'identifier, un traitement est appliqué pour isoler le contour, de préférence le contour des dents.

La figure 5 représente un exemple de première vue, avant traitement pour isoler le contour.

La partie gauche de la figure 7 représente un exemple de première vue, après traitement pour isoler le contour des dents.

**A l'étape 2),** on modifie le modèle historique pour simuler l'effet d'un évènement dentaire.

La modification du modèle historique peut notamment consister en un déplacement, une déformation ou une suppression du modèle élémentaire d'une ou plusieurs dents (« modèle de dent »), et/ou de la gencive, et/ou d'une ou des deux mâchoires, et/ou d'un appareil orthodontique.

La modification peut être effectuée manuellement, par un opérateur, de préférence par un professionnel de soins dentaires, de préférence encore un orthodontiste, de préférence à l'aide d'un ordinateur lui permettant de visualiser le modèle en cours de modification.

L'étape 2) conduit à un modèle historique qui représente une situation dentaire théorique.

Ce modèle peut avantageusement simuler des situations dentaires pour lesquelles des mesures ne sont pas disponibles. En particulier, il est possible de créer des modèles historiques correspondant à différents stades d'une pathologie rare.

**A l'étape 3),** on acquiert une vue du modèle historique modifié à l'étape 2), dite « deuxième vue », dans les mêmes conditions d'observation du modèle historique que celles ayant permis d'acquérir la première vue. Autrement dit, les représentations, sur les première et deuxième vues, des dents qui ne se sont pas déplacées entre les étapes 1) et 3) peuvent être mises en superposition parfaite, c'est-à-dire « en registre ».

Si la deuxième vue ne représente pas un contour, ou ne permet pas de l'identifier, un traitement est appliqué pour isoler le contour, de préférence le contour des dents.

La partie droite de la figure 7 représente un exemple de deuxième vue, après traitement pour isoler le contour des dents. Une comparaison des parties gauche et droite de la figure 7 permet de visualiser l'effet de l'évènement dentaire, en l'occurrence l'effet d'un traitement orthodontique au moyen d'un appareil avec arc et attaches.

On génère ainsi un couple, ou « enregistrement historique », constitué de la première vue et de la deuxième vue associée matérialisant l'application de l'évènement dentaire sur l'arcade représentée sur la première image. L'enregistrement historique est ajouté dans la base d'apprentissage historique.

La figure 2 représente un exemple d'enregistrement historique.

Dans un mode de réalisation, à l'étape 1), on acquiert plus de 10, plus de 100, plus de 1 000, plus de 10 000 premières vues dans des premières conditions d'observations à chaque fois différentes, puis, à l'étape 3), pour chaque première vue acquise dans des premières conditions d'observation, on acquiert une deuxième vue dans des deuxièmes conditions d'observation identiques aux dites premières conditions d'observation et on crée un enregistrement historique avec lesdites première et deuxième vues.

Cette procédure est équivalente à la réalisation, après l'étape 3), de plus de 10, plus de 100, plus de 1 000, plus de 10 000 cycles d'étapes 1) et 3), sans étape 2), en modifiant à chaque cycle les premières conditions d'observations. Autrement dit, on acquiert des première et deuxième vues en se déplaçant autour du modèle historique, notamment en tournant autour du modèle historique, et/ou en se rapprochant ou en s'éloignant, et/ou en modifiant la calibration de l'appareil d'acquisition virtuel permettant d'acquérir les premières et deuxièmes vues.

L'invention permet ainsi avantageusement de multiplier les enregistrements historiques avec un même modèle historique.

Ensuite, on change de modèle historique et on reprend à l'étape 1).

On constitue ainsi une base d'apprentissage historique comportant de préférence plus de 5 000, de préférence plus de 10 000, de préférence plus de 30 000, de préférence plus de 50 000, de préférence plus de 100 000 enregistrements historiques.

**A l'étape 4),** on entraine ensuite le réseau de neurones avec la base d'apprentissage historique. Un tel entrainement est bien connu de l'homme de l'art.

Il consiste classiquement à fournir l'ensemble desdites premières vues en entrée du réseau de neurones et l'ensemble desdites deuxièmes vues en sortie du réseau de neurones, en établissant pour chaque première vue une relation bijective avec la deuxième vue correspondante, c'est-à-dire appartenant au même enregistrement.

Par cet entrainement, le réseau de neurones apprend à transformer une vue d'entrée représentant un contour d'une arcade dentaire d'analyse (comme la première vue), en une vue de sortie représentant la même arcade, mais après que l'évènement dentaire a eu lieu.

Le réseau de neurones peut être en particulier choisi parmi les réseaux spécialisés dans la génération d'images, par exemple :
- Cycle-Consistent Adversarial Networks (2017)
- Augmented CycleGAN (2018)
- Deep Photo Style Transfer (2017)
- FastPhotoStyle (2018)
- pix2pix (2017)
- Style-Based Generator Architecture for GANs (2018)
- SRGAN (2018).

La liste ci-dessus n'est pas limitative.

Le réseau de neurones ainsi entraîné peut être utilisé pour simuler l'effet d'un évènement dentaire sur une arcade dentaire représentée sur une simple photo, suivant les étapes a) à e) décrites ci-dessous. Le réseau de neurones est alors qualifié de « réseau de neurones de transformation ».

### Simulation d'un évènement dentaire

**A l'étape a),** on crée une image d'origine en acquérant une photo avec un appareil photo, de préférence choisi parmi un téléphone portable, un appareil photo dit « connecté », une montre dite « intelligente », ou « smartwatch », une tablette ou un ordinateur personnel, fixe ou portable, comportant un système d'acquisition de photos. De préférence, l'appareil photo est un téléphone portable.

De préférence, lors de l'acquisition de la photo, l'appareil photo est écarté de l'arcade dentaire de plus de 5 cm, plus de 8 cm, voire plus de 10 cm, ce qui évite la condensation de vapeur d'eau sur l'optique de l'appareil photo et facilite la mise au point. En outre, de préférence, l'appareil photo, en particulier le téléphone portable, n'est pourvu d'aucune optique spécifique pour l'acquisition des photos, ce qui est notamment possible du fait de l'écartement de l'arcade dentaire lors de l'acquisition.

Pour faciliter l'acquisition de la photo, l'écarteur et l'appareil photo sont de préférence fixés sur un même support, ce qui permet de fixer leurs positions relatives. De préférence, le support est portable et doit être tenu à la main par le bénéficiaire lorsqu'il prend la photo.

De préférence, la photo est en couleurs, de préférence en couleurs réelles.

De préférence, l'acquisition de la photo est effectuée par le bénéficiaire, de préférence sans utilisation d'un support d'immobilisation de l'appareil photo, et notamment sans trépied.

La photo est de préférence une vue extra-orale, par exemple une vue correspondant à une photo qui aurait été prise face au patient, de préférence avec un écarteur.

L'écarteur peut présenter les caractéristiques des écarteurs conventionnels.

**A l'étape b),** le traitement de l'image d'origine a pour objectif de mettre en évidence, voire isoler, une information discriminante contenue dans l'image d'origine. L'utilisation de l'information discriminante améliore considérablement l'efficacité du réseau de neurones mis en œuvre à l'étape c).

De préférence, on traite l'image d'origine pour faire apparaître et de préférence isoler un contour.

De préférence, l'image d'origine est traitée de manière à ne sensiblement plus représenter qu'un contour. De préférence, le contour comprend le contour élémentaire de chaque dent représentée, voire est constitué de l'ensemble des contours élémentaire des dents.

Le contour peut aussi comprendre, voire être constitué du seul contour de l'ensemble des dents représentées. Ce mode de réalisation n'est cependant pas préféré.

L'homme de l'art sait comment traiter une photo ou une vue pour isoler un contour. Un tel traitement comporte par exemple l'application de masques ou de filtres bien connus, fournis avec des logiciels de traitement d'images. De tels traitements permettent par exemple de détecter les régions de fort contraste.

Ces traitements comprennent notamment une ou plusieurs des méthodes connues et préférées suivantes :
- application d'un filtre Canny, notamment pour rechercher des contours en utilisant l'algorithme de Canny ;
- application d'un filtre Sobel, notamment pour calculer des dérivées au moyen de l'opérateur étendu de Sobel ;
- application d'un filtre de Laplace, pour calculer le laplacien d'une image ;
- détection de tâches sur une image (« Blobdetector ») ;
- application d'un seuil (« Threshold ») pour appliquer un seuil fixe à chaque élément d'un vecteur ;
- redimensionnement, en utilisant des relations entre les zones de pixels (« Resize(Area) ») ou des interpolations bi-cubiques sur l'environnement des pixels ;
- érosion de l'image au moyen d'un élément spécifique structurant ;
- dilatation de l'image au moyen d'un élément spécifique structurant ;
- retouche, en particulier en utilisant des régions au voisinage de la zone restaurée ;
- application d'un filtre bilatéral ;
- application d'un flou gaussien ;
- application d'un filtre d'Otsu, pour rechercher le seuil qui minimise la variance intra-classes ;
- application d'un filtre A*, pour rechercher un chemin entre des points ;
- application d'un seuil adaptatif (« Adaptive Threshold ») pour appliquer un seuil adaptatif à un vecteur ;
- application d'un filtre d'égalisation d'un histogramme d'une image en nuances de gris en particulier ;
- détection de flou ("BlurDetection"), pour calculer l'entropie d'une image en utilisant son laplacien ;
- détection de contours (« FindContour ») d'une image binaire ;
- remplissage de couleurs ("FloodFill"), notamment pour remplir un élément connecté avec une couleur déterminée.

Les méthodes non limitatives suivantes, bien qu'elles ne soient pas préférées, peuvent être également mises en œuvre :
- application d'un filtre "MeanShift", de manière à trouver un objet sur une projection de l'image ;
- application d'un filtre « CLAHE », pour « Contrast Limited Adaptive Histogram Equalization » ;
- application d'un filtre « Kmeans", pour déterminer le centre de clusters et de groupes d'échantillons autour de clusters ;
- application d'un filtre DFT, de manière à effectuer une transformation de Fourier discrète, directe ou inverse d'un vecteur ;
- calcul de moments ;
- application d'un filtre « HuMoments » pour calculer des invariants de Hu invariants ;
- calcul de l'intégrale d'une image ;
- application d'un filtre Scharr, permettant de calculer une dérivée de l'image en mettant en œuvre un opérateur de Scharr ;
- recherche de l'enveloppe convexe de points ("ConvexHull") ;
- recherche de points de convexité d'un contour ("ConvexityDefects") ;
- comparaison de formes (« MatchShapes ») ;
- vérification si des points sont dans un contour (« PointPolygonTest ») ;
- détection des contours Harris (« CornerHarris ») ;
- la recherche des valeurs propres minimales de matrices de gradients, pour détecter les coins ("CornerMinEigenVal ») ;
- application d'une transformée de Hough pour trouver des cercles dans une image en nuances de gris (« HoughCircles ») ;
- "Active contour modeling" (traçage du contour d'un objet à partir d'une image 2D potentiellement « bruitée » ) ;
- calcul d'un champ de forces, appelées GVF ("gradient vector flow"), dans une partie de l'image ;
- classement en cascade (« CascadeClassification »).

Le traitement peut être également effectué au moyen d'un réseau de neurones entrainé à cet effet. Ce réseau de neurones est de préférence choisi parmi les réseaux spécialisés dans la localisation, et la détection d'objets dans une image, les « Object Detection Networks », par exemple
- R-CNN (2013)
- SSD (Single Shot MultiBox Detector : Object Detection network), Faster R-CNN (Faster Region-based Convolutional Network method : Object Detection network)
- Faster R-CNN (2015)
- SSD (2015).

La détermination des contours des dent peut être optimisée en suivant les enseignements de PCT/EP2015/074900 ou de FR1901755.

L'étape b) permet avantageusement d'obtenir une image très simple à traiter par un réseau de neurones.

**A l'étape c),** on présente en entrée du réseau de neurones de simulation l'image d'origine issue de l'étape b). Le réseau de neurones de simulation modifie alors l'image d'origine pour simuler l'effet de l'évènement dentaire sur l'arcade dentaire qui y est représentée.

Avantageusement, seule l'image d'origine a besoin d'être présentée à l'entrée du réseau de neurones de simulation. Aucune information tridimensionnelle, par exemple un modèle numérique tridimensionnel, n'a besoin d'être présentée au réseau de neurones de simulation. Par ailleurs, toute l'image d'origine peut être soumise au réseau de neurones de simulation. Il n'est pas nécessaire d'isoler un élément de cette image. Le réseau de neurones de simulation génère, à partir de l'image d'origine, une nouvelle image. Autrement dit, aucune partie de l'image d'origine n'est simplement copiée. N'importe quelle partie de l'image d'origine est susceptible d'être modifiée lors de la génération de la nouvelle image.

De préférence, le réseau de neurones de simulation a préalablement été entrainé en lui fournissant :
- en entrée, un ensemble d'images « d'entrée » représentant chacune l'information discriminante d'une image réaliste « d'entrée » respective représentant une arcade dentaire respective, par exemple d'une photo de cette arcade ou d'une vue hyperréaliste d'un modèle tridimensionnel de cette arcade, et
- en sortie, un ensemble d'images « de sortie », chaque image de sortie étant associée à une image d'entrée et représentant l'information discriminante d'une image réaliste « de sortie » respective représentant l'arcade dentaire représentée sur l'image hyperréaliste « d'entrée » associée, mais après occurrence d'un évènement dentaire, l'image réaliste de sortie étant par exemple une photo de cette arcade ou une vue hyperréaliste d'un modèle tridimensionnel de cette arcade.

Par « réaliste », on entend que la représentation de l'arcade est similaire à celle que pourrait observer une personne à l'œil nu, dans la réalité.

Par cet entrainement, le réseau de neurones de simulation apprend à transformer une image d'entrée en une image de sortie, et donc apprend à simuler l'évènement dentaire.

De préférence, l'information discriminante représentée sur les images d'entrée et de sortie est un contour.

Le réseau de neurones de simulation utilisé peut avoir notamment été entrainé suivant les étapes 1) à 4).

**A l'étape d),** optionnelle mais préférée, on modifie l'image modifiée obtenue à l'issue de l'étape c), pour qu'elle soit hyperréaliste, c'est-à-dire qu'elle semble être une photo.

Tous les moyens pour rendre hyperréaliste l'image modifiée sont possibles.

Des techniques de texturation d'images sont décrites dans l'article de Zhu, Jun-Yan, et al. "Unpaired image-to-image translation using cycle-consistent adversarial networks*. "*

De préférence, on utilise un réseau de neurones dit « de texturation » entrainé pour rendre hyperréalistes des images représentant des contours, comme l'image modifiée, et comportant de préférence les étapes d0) à d3) ci-dessous.

Le réseau de neurones de texturation peut être choisi dans la liste des réseaux de neurones présentée ci-dessus pour le réseau de neurones mis en œuvre dans le procédé d'entrainement selon l'invention. Le réseau de neurones de texturation peut être en particulier choisi parmi les réseaux spécialisés dans la génération d'images, par exemple :
- Cycle-Consistent Adversarial Networks (2017)
- Augmented CycleGAN (2018)
- Deep Photo Style Transfer (2017)
- FastPhotoStyle (2018)
- pix2pix (2017)
- Style-Based Generator Architecture for GANs (2018)
- SRGAN (2018).

Il n'est cependant pas limité à cette liste.

**A l'étape d0)** pour chaque photo, dite « photo de texturation », représentant une arcade dentaire d'un ensemble comportant plus de 1 000 photos de texturation, on traite la photo de texturation, de préférence comme à l'étape b), de manière à obtenir une image, dite « image de texturation », représentant un contour.

**A l'étape d1),** on crée une base d'apprentissage dite « de texturation » constituée d'enregistrements dits « de texturation », chaque enregistrement de texturation comportant :
- une photo de texturation représentant une arcade dentaire, et
- l'image de texturation obtenue, lors d'une étape d0) préalable à l'étape d1), par traitement de ladite photo de texturation à l'étape d0).

**A l'étape d2),** on entraine le réseau de neurones de texturation au moyen de la base d'apprentissage de texturation. Un tel entrainement est bien connu de l'homme de l'art.

Il consiste classiquement à fournir l'ensemble desdites images de texturation en entrée du réseau de neurones de texturation et l'ensemble desdites photos de texturation en sortie du réseau de neurones de texturation, en informant le réseau de neurones de texturation de la photo de texturation qui correspond à chaque image de texturation.

Par cet entrainement, le réseau de neurones de texturation apprend à transformer une image représentant un contour d'arcade dentaire, comme l'image modifiée, en une image hyperréaliste.

**A l'étape d3),** on soumet l'image modifiée au réseau de neurones de texturation entrainé. Le réseau de neurones de texturation la transforme en une image hyperréaliste.

La figure 3 représente un exemple de photo acquise à l'étape a) (à gauche) et d'image modifiée obtenue à l'issue de l'étape d) (à droite). On peut constater l'effet de l'évènement dentaire sur la position de certaines dents.

**A l'étape e),** l'image modifiée, de préférence rendue hyperréaliste, peut être présentée, notamment au bénéficiaire, de préférence sur un écran, de préférence sur un écran d'un téléphone portable, d'une tablette, d'un ordinateur portable, ou d'un casque de réalité virtuelle. L'écran peut être également la glace d'un miroir.

Le procédé de génération et le procédé d'entrainement sont mis en œuvre au moyen d'un ordinateur. Classiquement, un ordinateur comporte en particulier un processeur, une mémoire, une interface homme-machine, comportant classiquement un écran, un module de communication par internet, par WIFI, par Bluetooth^{®} ou par le réseau téléphonique. Un logiciel configuré pour mettre en œuvre le procédé de l'invention considéré est chargé dans la mémoire de l'ordinateur.

L'ordinateur peut être également connecté à une imprimante.

Dans un mode de réalisation, l'interface homme-machine permet de communiquer à l'ordinateur :
- un instant de simulation lorsque l'évènement dentaire comporte un écoulement de temps entre l'instant d'acquisition et ledit instant de simulation, et/ou
- un paramètre d'un traitement appliqué au bénéficiaire ; et/ou
- un paramètre d'un appareil orthodontique porté par le bénéficiaire, par exemple relatif à la classe et/ou à la conformation de l'appareil orthodontique ; et/ou
- un paramètre fonctionnel du bénéficiaire, en particulier un paramètre neurofonctionnel, comme la facilité à respirer, à déglutir ou à fermer la bouche ; et/ou
- un paramètre anatomique du bénéficiaire autre que les paramètres de positionnement de ses dents, comme l'agencement et/ou la structure de tissus osseux (notamment des mâchoires) et/ou de tissus alvéodentaires et/ou de tissus mous (notamment les gencives et/ou les freins et/ou la langue et/ou les joues) ; et/ou
- l'âge, ou une tranche d'âge, et/ou le sexe dudit bénéficiaire.

Il est ainsi possible à l'ordinateur de choisir un réseau de neurones de simulation entrainé en conséquence.

Par exemple, il est possible de choisir un réseau de neurones de simulation entrainé pour simuler un déplacement des dents avec un appareil orthodontique à arc et attaches, pour un homme de 30 à 40 ans, ayant un tissu osseux « normal ».

Le procédé permet en particulier de simuler l'effet de différents traitements orthodontiques, ce qui facilite le choix du traitement le plus adapté aux besoins ou aux souhaits du bénéficiaire.

De préférence, l'interface homme-machine comporte un écran présentant un champ de saisie de l'instant de simulation.

Dans un mode de réalisation, l'interface homme-machine permet de sélectivement afficher ou ne pas afficher à l'écran un appareil orthodontique porté par le bénéficiaire.

### Exemples

### Simulation d'une situation dentaire passée ou future

Dans un mode de réalisation, le bénéficiaire prend l'image d'origine, par exemple avec son téléphone portable (étape a)), et un ordinateur, intégré dans le téléphone portable ou avec lequel le téléphone portable peut communiquer, met en œuvre les étapes b) à e). L'image modifiée est de préférence présentée sur l'écran du téléphone portable.

De préférence, l'ordinateur est intégré dans le téléphone portable, ce qui permet au bénéficiaire de mettre en œuvre le procédé de génération selon l'invention de manière totalement autonome.

Le bénéficiaire peut ainsi demander très facilement une simulation de situation dentaire, sans même devoir se déplacer, à partir d'une ou de préférence plusieurs photos de ses dents.

En particulier, la situation dentaire peut être simulée à un instant de simulation passé ou futur. L'instant de simulation peut être par exemple antérieur ou postérieur à l'instant d'acquisition de l'image d'origine, par exemple de plus de 1 jour, 10 jours ou 100 jours à l'instant d'acquisition.

Dans un cas particulier, l'évènement dentaire est l'écoulement du temps dans le cadre d'un traitement, par exemple d'un traitement orthodontique pendant lequel le bénéficiaire porte un appareil orthodontique.

De préférence, le procédé comporte une étape d). L'image modifiée présentée apparaît alors comme une photo qui aurait été prise à l'instant de simulation. Elle peut être présentée au bénéficiaire afin de lui montrer, par exemple, sa situation dentaire future ou passée, et ainsi le motiver à observer le traitement.

Dans un cas particulier, l'évènement dentaire est l'écoulement du temps dans le cadre d'un traitement orthodontique pendant lequel le bénéficiaire ne respecte pas les prescriptions médicales, par exemple ne porte pas correctement son appareil orthodontique. La présentation d'une image modifiée photoréaliste permet ainsi de visualiser l'effet d'une mauvaise observance.

Un programme selon l'invention peut être en particulier utilisé pour simuler :
- l'effet d'un ou plusieurs appareils orthodontiques sur les dents du bénéficiaire, notamment afin de choisir celui qui lui convient le mieux ;
- l'effet d'un arrêt, temporaire ou définitif, d'un traitement en cours ;
- l'effet d'une application d'une consigne ;
- l'effet d'un traitement, thérapeutique ou non thérapeutique.

Notamment, le programme peut être utilisé, en particulier à des fins pédagogiques, pour visualiser l'effet d'une modification de la fréquence et/ou de la durée et/ou de la technique de brossage des dents, ou l'effet d'un retard dans un changement de gouttière orthodontique et/ou d'un retard pour prendre rendez-vous chez le professionnel des soins dentaires.

### Simulation dynamique

Dans un mode de réalisation, les étapes a) à e) sont répétées en boucle, avec des images d'origine acquises successivement. De préférence, chaque cycle dure moins de 5 s, de préférence moins de 2 s, de préférence moins de 1 s. A l'étape a), on utilise de préférence une caméra.

Le bénéficiaire peut par exemple voir les images modifiées sur un miroir équipé d'une caméra, dans lequel il se regarde. De préférence, les images modifiées sont présentées en registre avec les images réfléchies par le miroir, c'est-à-dire que le bénéficiaire voit les images modifiées comme si elles étaient obtenues par réflexion. Il a donc l'impression de s'observer à l'instant de simulation.

De préférence, entre deux cycles d'étapes a) à e), on peut modifier l'instant de simulation, par exemple en modifiant la position d'un curseur représenté sur l'écran. De préférence, l'écran est un écran tactile et on modifie l'instant de simulation par interaction, de préférence par glissement, d'un doigt sur ledit écran.

### Evènement à effet instantané

Dans un cas particulier, l'évènement dentaire a un effet immédiat que l'on souhaite visualiser.

Par exemple l'évènement dentaire est la pose d'un appareil orthodontique. Le procédé permet ainsi d'intégrer dans l'image d'origine une représentation d'un appareil orthodontique, ou de modifier un appareil orthodontique représenté sur l'image d'origine, ou de supprimer un appareil orthodontique représenté sur l'image d'origine, sans modification de la position des dents.

Le réseau de neurones de simulation est entrainé pour créer une image modifiée à partir de l'image d'origine qui lui est fournie. Ce procédé est donc tout à fait différent d'un procédé dans lequel, par exemple, on ajoute à une image un élément, par exemple une représentation d'un appareil orthodontique existant. En effet, pour intégrer dans l'image d'origine une représentation d'un appareil orthodontique, le réseau de neurones de simulation crée cette représentation. Cette représentation n'est donc pas la reproduction d'un appareil orthodontique réel ou d'un modèle tridimensionnel d'un appareil orthodontique réel, mais est générée par le réseau de neurones de simulation de manière artificielle, en même temps que le reste de l'image.

De manière surprenante, la représentation de l'appareil orthodontique est très réaliste et permet une bonne simulation pour le bénéficiaire. Notamment, l'entrainement du réseau de neurones de simulation lui apprend à représenter l'appareil orthodontique dans le contexte de l'image d'origine, avec le contraste, la netteté, les ombres et les reflets correspondant. La simulation est donc beaucoup plus réaliste que le simple ajout, dans une image représentant l'arcade dentaire, d'une représentation préexistante d'un appareil orthodontique.

La modification de l'image d'origine par le réseau de neurones peut conduire à des modifications d'autres zones de l'image d'origine que la zone de représentation de l'appareil orthodontique. Ces différences, qui pourraient être préjudiciables si l'image modifiée était utilisée pour intervenir sur les dents, par exemple pour guider un dentiste lors d'une opération de fraisage, ne le sont pas lorsque l'image modifiée est destinée à être présentée au bénéficiaire. Les performances des réseaux de neurones peuvent même rendre sensiblement impossible la détection de différence en dehors de la zone dans laquelle l'appareil orthodontique a été représenté.

Comme cela apparaît clairement à présent, l'invention permet à un bénéficiaire de simuler l'effet d'un évènement dentaire sur les dents de ses arcades dentaires, sans qu'il ait besoin de réaliser un scan de cette arcade dentaire. Dans un mode de réalisation préféré, toute personne équipée d'un téléphone portable peut avantageusement réaliser une telle simulation.

Bien entendu, l'invention n'est pas limitée aux modes de réalisation décrits ci-dessus et représentés.

En particulier, le bénéficiaire n'est pas limité à un être humain. Un programme selon l'invention peut être utilisé pour un autre animal.

Par ailleurs, le procédé d'entrainement ne comporte pas nécessairement les étapes 1) à 4), qui sont cependant préférées.

Un réseau de neurones, en particulier le réseau de neurones de simulation, peut être par exemple entrainé par la mise en œuvre d'un procédé comportant, pour un ensemble d'individus comportant de préférence plus de 100, de préférence plus de 1 000, de préférence plus de 10 000 individus, les étapes suivantes :
l') acquisition d'une photo d'une arcade dentaire d'un individu et traitement de ladite photo de manière à obtenir une première image représentant un contour de l'arcade dentaire représentée sur la photo ;
2') génération d'un modèle tridimensionnel numérique représentant ladite arcade dentaire après survenue de l'évènement dentaire ;
3') acquisition d'une vue dudit modèle cadrée avec la photo et, si ladite vue ne représente pas un contour, traitement de la vue de manière qu'elle représente un contour de l'arcade dentaire représentée ;
puis, avec l'ensemble des premières images et vues acquises pour l'ensemble des individus :
4') introduction, en entrée et en sortie du réseau de neurones, desdites photos et vues, respectivement, de manière à entrainer ledit réseau de neurones à transformer une vue d'entrée représentant une arcade dentaire d'analyse en une vue de sortie représentant ladite arcade dentaire d'analyse après application dudit évènement dentaire.

A l'étape 2'), la génération du modèle représentant l'arcade dentaire après survenue de l'évènement dentaire peut résulter de
- la génération d'un modèle avant survenue de l'évènement dentaire, par exemple sensiblement au même moment que celui auquel la photo a été acquise (étape 1')), puis
- la simulation de l'évènement dentaire sur ledit modèle, comme décrit pour l'étape 2).

A l'étape 3'), la vue doit être cadrée avec la photo. De préférence, on recherche des conditions d'observation du modèle qui correspondent au mieux (« *best fit* ») aux conditions d'acquisition de la photo. Autrement dit, on recherche une position, une orientation et une calibration d'un appareil d'acquisition virtuel permettant d'observer le modèle selon une vue dans laquelle la représentation des dents qui ne se sont pas déplacées lors de l'évènement dentaire est superposable en registre avec la représentation desdites dents sur la photo.

## Revendications

1. Programme d'ordinateur comprenant des instructions de code de programme pour l'exécution d'un procédé de génération d'une image d'une arcade dentaire d'un bénéficiaire, dite « image modifiée », ledit procédé comportant les étapes successives suivantes :
a) à un instant d'acquisition, acquisition d'une photo représentant ladite arcade dentaire, dite « image d'origine » ;
b) traitement de l'image d'origine pour qu'elle représente une information discriminante ;
c) soumission de l'image d'origine issue de l'étape b) en entrée d'un réseau de neurones, dit « réseau de neurones de simulation », entrainé pour, à partir de l'image d'origine, simuler, sur l'image d'origine, l'effet d'un évènement dentaire, afin d'obtenir l'image modifiée, l'évènement dentaire étant choisi parmi un écoulement de temps dans le cadre d'un traitement orthodontique ou non orthodontique, dans le cadre d'une pathologie ou dans le cadre d'un bruxisme, une pose d'un organe dentaire sur l'arcade dentaire, un écoulement du temps en l'absence de traitement, et les combinaisons de ces évènements dentaires ;
d) de préférence, traitement de l'image modifiée pour la rendre hyperréaliste ;
e) de préférence,
- présentation de l'image modifiée ; et/ou
- sélection d'un appareil orthodontique en fonction de l'image modifiée.

2. Programme d'ordinateur selon l'une quelconque des revendications précédentes, dans lequel l'information discriminante est choisie dans le groupe constitué par un information de contour, une information de couleur, une information de densité, une information de distance, une information de brillance, une information de saturation, une information sur les reflets et des combinaisons de ces informations.

3. Programme d'ordinateur selon la revendication précédente, dans lequel la représentation de l'information discriminante est un contour de l'arcade dentaire.

4. Programme d'ordinateur selon l'une quelconque des revendications précédentes, dans lequel le réseau de neurones de simulation est entrainé au moyen d'un procédé d'entrainement comportant, pour chacun d'une pluralité de modèles tridimensionnels numériques d'arcades dentaires « historiques », dit « modèles historiques », les étapes successives suivantes :
1) acquisition d'une première vue du modèle historique dans des premières conditions d'observations et, si ladite première vue ne représente pas une information discriminante de l'arcade dentaire historique, dite « première information discriminante », traitement de la première vue de manière qu'elle représente ladite première information discriminante ;
2) modification du modèle historique, de manière à reproduire l'effet dudit évènement dentaire sur ladite arcade dentaire historique ;
3) acquisition d'une deuxième vue du modèle historique dans des deuxièmes conditions d'observations identiques aux premières conditions d'observations et, si ladite deuxième vue ne représente pas ladite information discriminante de l'arcade dentaire historique, dite « deuxième information discriminante », traitement de la deuxième vue de manière qu'elle représente ladite deuxième information discriminante ;
puis, avec l'ensemble des premières et deuxièmes vues acquises pour l'ensemble des modèles historiques :
4) introduction desdites premières et deuxièmes vues, en entrée et en sortie du réseau de neurones de simulation, respectivement, de manière à entrainer ledit réseau de neurones de simulation à transformer une vue d'entrée représentant une arcade dentaire d'analyse en une vue de sortie représentant ladite arcade dentaire d'analyse après application dudit évènement dentaire.

5. Programme d'ordinateur selon la revendication immédiatement précédente, dans lequel le réseau de neurones de simulation est entrainé au moyen d'un procédé d'entrainement comportant, pour chacun d'une pluralité de modèles tridimensionnels numériques d'arcades dentaires « historiques », dit « modèles historiques », les étapes successives suivantes :
1) acquisition d'une première vue du modèle historique dans des premières conditions d'observations et, si ladite première vue ne représente pas un contour de l'arcade dentaire historique, dit « premier contour », traitement de la première vue de manière qu'elle représente un premier contour ;
2) modification du modèle historique, de manière à reproduire l'effet dudit évènement dentaire sur ladite arcade dentaire historique ;
3) acquisition d'une deuxième vue du modèle historique dans des deuxièmes conditions d'observations identiques aux premières conditions d'observations et, si ladite deuxième vue ne représente pas un contour de l'arcade dentaire historique, dit « deuxième contour », traitement de la deuxième vue de manière qu'elle représente un deuxième contour ;
puis, avec l'ensemble des premières et deuxièmes vues acquises pour l'ensemble des modèles historiques :
4) introduction desdites premières et deuxièmes vues, en entrée et en sortie du réseau de neurones de simulation, respectivement, de manière à entrainer ledit réseau de neurones de simulation à transformer une vue d'entrée représentant une arcade dentaire d'analyse en une vue de sortie représentant ladite arcade dentaire d'analyse après application dudit évènement dentaire.

6. Programme d'ordinateur selon l'une quelconque des deux revendications immédiatement précédentes, dans lequel
- à l'étape 1), on acquiert plus de 10 premières vues dans des premières conditions d'observations à chaque fois différentes, puis,
- à l'étape 3), pour chaque première vue acquise dans des premières conditions d'observation, on acquiert une deuxième vue dans des deuxièmes conditions d'observation identiques aux dites premières conditions d'observation et on crée un enregistrement historique avec lesdites première et deuxième vues.

7. Programme d'ordinateur selon l'une quelconque des revendications précédentes, dans lequel, à l'étape a), on acquiert la photo de manière extra-orale, avec un téléphone portable, le bénéficiaire portant un écarteur dentaire.

8. Programme d'ordinateur selon l'une quelconque des revendications précédentes, comportant une étape d) de traitement de l'image modifiée pour la rendre hyperréaliste, l'étape d) comportant les étapes suivantes :
d0) pour chaque photo, dite « photo de texturation », représentant une arcade dentaire d'un ensemble comportant plus de 1 000 photos de texturation, traitement de la photo de texturation, de préférence comme à l'étape b), de manière à obtenir une image, dite « image de texturation », représentant un contour ;
d1) création d'une base d'apprentissage dite « de texturation » constituée d'enregistrements, dits « enregistrements de texturation », chaque enregistrement de texturation comportant une photo de texturation et l'image de texturation obtenue par traitement de ladite photo de texturation à l'étape d0) ;
d2) entrainement d'un réseau de neurones, dit « réseau de neurones de texturation », au moyen de la base d'apprentissage de texturation ;
d3) soumission de l'image modifiée au réseau de neurones de texturation entrainé, de manière à obtenir une image modifiée hyperréaliste.

9. Programme d'ordinateur selon l'une quelconque des revendications précédentes, dans lequel l'évènement dentaire est un écoulement de temps depuis l'instant d'acquisition jusqu'à un instant de simulation antérieur ou postérieur à l'instant d'acquisition de plus de 1 jour, et dans lequel
à l'étape e), l'image modifiée est présentée au bénéficiaire afin de lui montrer une situation dentaire déterminée audit instant de simulation.

10. Programme d'ordinateur selon l'une quelconque des revendications précédentes, dans lequel avant l'étape c), on détermine l'évènement dentaire en précisant un instant de simulation, et/ou un paramètre d'un traitement appliqué au bénéficiaire et/ou un paramètre d'un appareil orthodontique porté par le bénéficiaire, et/ou un paramètre fonctionnel du bénéficiaire, et/ou un paramètre anatomique du bénéficiaire autre que les paramètres de positionnement de ses dents, et/ou un âge, ou une tranche d'âge, et/ou un sexe dudit bénéficiaire.

11. Programme d'ordinateur selon l'une quelconque des revendications précédentes, dans lequel les étapes a) à e) sont répétées en boucle, avec des images d'origine acquises successivement, chaque cycle durant moins de 5 s.

12. Programme d'ordinateur selon l'une quelconque des revendications précédentes, dans lequel, à l'étape c), on ne soumet que l'image d'origine en entrée du réseau de neurones de simulation.

## Patentansprüche

1. Computerprogramm, umfassend Programmcodeanweisungen zum Ausführen eines Verfahrens zum Generieren eines Bildes eines Zahnbogens eines Behandelten, des sogenannten "modifizierten Bildes", wobei das Verfahren die folgenden aufeinanderfolgenden Schritte aufweist:
a) zu einem Erfassungszeitpunkt, Erfassen eines Fotos, das den Zahnbogen darstellt, das sogenannte "Originalbild";
b) Verarbeiten des Originalbildes, so dass es eine unterscheidende Information darstellt;
c) Übermitteln des Originalbildes aus Schritt b) am Eingang eines Neuronennetzes, des sogenannten "Simulationsneuronennetzes", das trainiert ist, anhand des Originalbildes die Auswirkungen eines zahnmedizinischen Ereignisses auf das Originalbild zu simulieren, um das modifizierte Bild zu erhalten, wobei das zahnmedizinische Ereignis aus einem Zeitablauf im Rahmen einer kieferorthopädischen oder nicht kieferorthopädischen Behandlung, im Rahmen einer Pathologie oder im Rahmen eines Bruxismus, eines Einsetzens eines Zahnersatzes in den Zahnbogen, eines Zeitablaufs ohne Behandlung und der Kombinationen dieser zahnmedizinischen Ereignisse ausgewählt ist;
d) vorzugsweise Verarbeiten des modifizierten Bildes, um es hyperrealistisch zu machen;
e) vorzugsweise
- Präsentieren des modifizierten Bildes; und/oder
- Auswählen einer kieferorthopädischen Einrichtung in Abhängigkeit des modifizierten Bildes.

2. Computerprogramm nach einem der vorstehenden Ansprüche, wobei die unterscheidende Information aus der Gruppe ausgewählt ist, die aus einer Konturinformation, einer Farbinformation, einer Dichteinformation, einer Abstandsinformation, einer Helligkeitsinformation, einer Sättigungsinformation, einer Reflexionsinformation und den Kombinationen dieser Informationen besteht.

3. Computerprogramm nach dem vorstehenden Anspruch, wobei die Darstellung der unterscheidenden Information eine Kontur des Zahnbogens ist.

4. Computerprogramm nach einem der vorstehenden Ansprüche, wobei das Simulationsneuronennetz mittels eines Trainingsverfahrens trainiert wird, das für jedes einer Vielzahl von dreidimensionalen numerischen Modellen "historischer" Zahnbögen, sogenannten "historischen Modellen", die folgenden aufeinanderfolgenden Schritte aufweist:
1) Erfassen einer ersten Ansicht des historischen Modells unter ersten Beobachtungsbedingungen und, falls diese erste Ansicht keine unterscheidende Information des historischen Zahnbogens darstellt, die sogenannte "erste unterscheidende Information", Verarbeiten der ersten Ansicht, sodass sie diese erste unterscheidende Information darstellt;
2) Modifizieren des historischen Modells, um die Auswirkung des zahnmedizinischen Ereignisses auf den historischen Zahnbogen zu reproduzieren;
3) Erfassen einer zweiten Ansicht des historischen Modells unter zweiten mit den ersten Beobachtungsbedingungen identischen Beobachtungsbedingungen und, falls diese zweite Ansicht nicht die unterscheidende Information des historischen Zahnbogens darstellt, die sogenannte "zweite unterscheidende Information", Verarbeiten der zweiten Ansicht, sodass sie diese zweite unterscheidende Information darstellt;
anschließend mit allen ersten und zweiten Ansichten, die für alle historischen Modelle erfasst wurden:
4) Einführen dieser ersten und zweiten Ansichten am Eingang bzw. Ausgang des Simulationsneuronennetzes, um das Simulationsneuronennetz so zu trainieren, dass es eine Eingangsansicht, die einen zu analysierenden Zahnbogen darstellt, in eine Ausgangsansicht umwandelt, die den zu analysierenden Zahnbogen nach Anwendung des zahnmedizinischen Ereignisses darstellt.

5. Computerprogramm nach dem unmittelbar vorstehenden Anspruch, bei dem das Simulationsneuronennetz mittels eines Trainingsverfahrens trainiert wird, das für jedes einer Vielzahl von dreidimensionalen numerischen Modellen "historischer" Zahnbögen, sogenannten "historischen Modellen", die folgenden aufeinanderfolgenden Schritte aufweist:
1) Erfassen einer ersten Ansicht des historischen Modells unter ersten Beobachtungsbedingungen und, falls diese erste Ansicht keine Kontur des historischen Zahnbogens darstellt, die sogenannte "erste Kontur", Verarbeiten der ersten Ansicht, sodass sie eine erste Kontur darstellt;
2) Modifizieren des historischen Modells, um die Auswirkung des zahnmedizinischen Ereignisses auf den historischen Zahnbogen zu reproduzieren;
3) Erfassen einer zweiten Ansicht des historischen Modells unter mit den ersten Beobachtungsbedingungen identischen zweiten Beobachtungsbedingungen und, falls diese zweite Ansicht keine Kontur des historischen Zahnbogens darstellt, die sogenannte "zweite Kontur", Verarbeiten der zweiten Ansicht, sodass sie eine zweite Kontur darstellt;
anschließend mit allen ersten und zweiten Ansichten, die für alle historischen Modelle erfasst wurden:
4) Einführen dieser ersten und zweiten Ansichten am Eingang bzw. Ausgang des Simulationsneuronennetzes, um das Simulationsneuronennetz so zu trainieren, dass es eine Eingangsansicht, die einen zu analysierenden Zahnbogen darstellt, in eine Ausgangsansicht umwandelt, die den zu analysierenden Zahnbogen nach Anwendung des zahnmedizinischen Ereignisses darstellt.

6. Computerprogramm nach einem der beiden unmittelbar vorstehenden Ansprüche, wobei
- in Schritt 1) mehr als 10 erste Ansichten unter ersten, jedes Mal unterschiedlichen Beobachtungsbedingungen erfasst werden, anschließend
- in Schritt 3) für jede erste Ansicht, die unter ersten Beobachtungsbedingungen erfasst wurde, eine zweite Ansicht unter mit den ersten Beobachtungsbedingungen identischen zweiten Beobachtungsbedingungen erfasst wird, und eine historische Aufzeichnung mit der ersten und der zweiten Ansicht erstellt wird.

7. Computerprogramm nach einem der vorstehenden Ansprüche, wobei in Schritt a) das Foto extraoral mit einem Mobiltelefon erfasst wird, wobei der Behandelte einen Zahnspreizer trägt.

8. Computerprogramm nach einem der vorstehenden Ansprüche, mit einem Schritt d) zum Verarbeiten des modifizierten Bildes, um es hyperrealistisch zu machen, wobei Schritt d) die folgenden Schritte aufweist:
d0) für jedes Foto, das sogenannte "Texturierungsfoto", das einen Zahnbogen aus einem Satz mit mehr als 1.000 Texturierungsfotos darstellt, Verarbeiten des Texturierungsfotos, vorzugsweise wie in Schritt b), um ein Bild, das sogenannte "Texturierungsbild", das eine Kontur darstellt, zu erhalten;
d1) Erstellen einer sogenannten "Texturierungs"-Lernbasis, die aus Aufzeichnungen, den sogenannten "Texturierungsaufzeichnungen", besteht, wobei jede Texturierungsaufzeichnung ein Texturierungsfoto und das durch das Verarbeiten des Texturierungsfotos in Schritt d0) erhaltene Texturierungsbild aufweist;
d2) Trainieren eines Neuronennetzes, des sogenannten "Texturierungsneuronennetzes", mittels der Texturierungslernbasis;
d3) Übermitteln des modifizierten Bildes an das trainierte Texturierungsneuronennetz, um ein hyperrealistisches modifiziertes Bild zu erhalten.

9. Computerprogramm nach einem der vorstehenden Ansprüche, wobei das zahnmedizinische Ereignis ein Zeitablauf vom Erfassungszeitpunkt bis zu einem Simulationszeitpunkt vor oder nach dem Erfassungszeitpunkt von mehr als 1 Tag ist und wobei
in Schritt e) das modifizierte Bild dem Behandelten präsentiert wird, um ihm eine bestimmte zahnmedizinische Situation zum Zeitpunkt der Simulation zu zeigen.

10. Computerprogramm nach einem der vorstehenden Ansprüche, wobei vor Schritt c) das zahnmedizinische Ereignis durch Angeben eines Simulationszeitpunkts und/oder eines Parameters einer beim Behandelten durchgeführten Behandlung und/oder eines Parameters einer vom Behandelten getragenen kieferorthopädischen Einrichtung und/oder eines Funktionsparameters des Behandelten und/oder eines anatomischen Parameters des Behandelten, die nicht die Positionierungsparameter seiner Zähne sind, und/oder eines Alters, oder einer Altersgruppe und/oder eines Geschlechts dieses Behandelten bestimmt wird.

11. Computerprogramm nach einem der vorstehenden Ansprüche, wobei die Schritte a) bis e) in einer Schleife wiederholt werden, wobei Originalbilder nacheinander erfasst werden und jeder Zyklus weniger als 5 s dauert.

12. Computerprogramm nach einem der vorstehenden Ansprüche, wobei in Schritt c) nur das Originalbild am Eingang des Simulationsneuronennetzes übermittelt wird.

## Claims

1. A computer program comprising program code instructions for executing a method for generating an image of a dental arch of a beneficiary, referred to as a "modified image", said method comprising the following successive steps:
a) at an acquisition moment, acquiring a photo depicting said dental arch, referred to as the "original image";
b) processing the original image so that it depicts discriminating information;
c) submitting the original image from step b) as input to a neural network, called a "simulation neural network", trained to simulate, from the original image, the effect of a dental event on the original image, in order to obtain the modified image, the dental event being chosen from a stretch of time in the context of orthodontic or non-orthodontic treatment, in the context of a pathology or in the context of bruxism, a placement of a dental organ on the dental arch, a stretch of time in the absence of treatment, and combinations of these dental events;
d) preferably, processing the modified image to make it hyperrealistic;
e) preferably,
- presenting the modified image; and/or
- selecting an orthodontic appliance based on the modified image.

2. The computer program according to any one of the preceding claims, wherein the discriminating information is selected from the group consisting of outline information, color information, density information, distance information, brightness information, saturation information, reflection information and combinations thereof.

3. The computer program according to the preceding claim, wherein the depiction of the discriminating information is a outline of the dental arch.

4. The computer program according to any one of the preceding claims, wherein the simulation neural network is trained by means of a training method comprising, for each of a plurality of digital three-dimensional models of "historical" dental arches, known as "historical models", the following successive steps:
1) acquiring a first view of the historical model under first observation conditions and, if said first view does not depict discriminating information of the historical dental arch, known as "first discriminating information", processing the first view so that it depicts said first discriminating information;
2) modifying the historical model, so as to reproduce the effect of said dental event on said historical dental arch;
3) acquiring a second view of the historical model under second observation conditions identical to the first observation conditions and, if said second view does not depict said discriminating information of the historical dental arch, known as "second discriminating information", processing the second view so that it depicts said second discriminating information;
then, with the set of first and second views acquired for all the historical models:
4) introducing said first and second views, as input and output of the simulation neural network, respectively, so as to train said simulation neural network to transform an input view depicting an analysis dental arch into an output view showing said analysis dental arch after applying said dental event.

5. The computer program according to the immediately preceding claim, wherein the simulation neural network is trained by means of a training method comprising, for each of a plurality of digital three-dimensional models of "historical" dental arches, known as "historical models", the following successive steps:
1) acquiring a first view of the historical model under first observation conditions and, if said first view does not depict an outline of the historical dental arch, known as "first outline", processing the first view so that it depicts said first outline;
2) modifying the historical model, so as to reproduce the effect of said dental event on said historical dental arch;
3) acquiring a second view of the historical model under second observation conditions identical to the first observation conditions and, if said second view does not depict said outline of the historical dental arch, known as "second outline", processing the second view so that it depicts said second outline;
then, with the set of first and second views acquired for all the historical models:
4) introducing said first and second views, as input and output of the simulation neural network, respectively, so as to train said simulation neural network to transform an input view depicting an analysis dental arch into an output view showing said analysis dental arch after applying said dental event.

6. The computer program according to any one of the two immediately preceding claims, wherein
- in step 1), more than 10 first views are acquired under different first observation conditions, then,
- in step 3), for each first view acquired under first observation conditions, a second view is acquired under second observation conditions identical to said first observation conditions, and a historical record is created with said first and second views.

7. The computer program according to any of the preceding claims, wherein, in step a), the photo is acquired extra-orally, using a cell phone, with the recipient wearing a dental retractor.

8. The computer program according to any one of the preceding claims, comprising a step d) of processing the modified image to make it hyperrealistic, step d) comprising the following steps:
d0) for each photo, known as a "texturing photo", showing a dental arch of a set comprising more than 1,000 texturing photos, processing the texturing photo, preferably as in step b), so as to obtain an image, known as a "texturing image", depicting a outline ;
d1) creating a "texturing" learning base consisting of records, known as "texturing records", each texturing record comprising a texturing photo and the texturing image obtained by processing said texturing photo in step d0);
d2) training a neural network, known as a "texturing neural network", using the texturing learning base;
d3) submitting the modified image to the trained texturing neural network, so as to obtain a hyperrealistic modified image.

9. The computer program according to any one of the preceding claims, wherein the dental event is a stretch of time from the acquisition moment to a simulation moment earlier or later than the acquisition moment by more than 1 day, and wherein
in step e), the modified image is presented to the beneficiary to show a dental situation determined at said simulation moment.

10. The computer program according to any one of the preceding claims, wherein prior to step c), the dental event is determined by specifying a simulation moment, and/or a parameter of a treatment applied to the beneficiary, and/or a parameter of an orthodontic appliance worn by the beneficiary, and/or a functional parameter of the beneficiary, and/or an anatomical parameter of the beneficiary other than the positioning parameters of their teeth, and/or an age, or an age range, and/or a sex of said beneficiary.

11. The computer program according to any of the preceding claims, wherein steps a) to e) are repeated in a loop, with original images acquired successively, each cycle lasting less than 5 s.

12. The computer program according to any of the preceding claims, wherein, in step c), only the original image is input to the simulation neural network.
